(19) Europäisches Patentamt
European Patent Office
Office européen des brevets

(11) Veröffentlichungsnummer: **0 011 273**
**A1**

(12) # EUROPÄISCHE PATENTANMELDUNG

(21) Anmeldenummer: **79104453.0**

(22) Anmeldetag: **12.11.79**

(51) Int. Cl.³: **C 07 C 47/42**
A 61 K 7/46, C 11 D 3/50
C 11 D 9/44, C 07 C 45/50

(30) Priorität: **16.11.78 DE 2849642**

(43) Veröffentlichungstag der Anmeldung:
**28.05.80 Patentblatt 80/11**

(84) Benannte Vertragsstaaten:
**BE CH DE FR GB IT SE**

(71) Anmelder: **Henkel Kommanditgesellschaft auf Aktien**
**Postfach 1100**
**D-4000 Düsseldorf 1(DE)**

(72) Erfinder: **Hagen, Jens, Dr.**
**Dieselstrasse 5**
**D-6834 Ketsch(DE)**

(72) Erfinder: **Bruns, Klaus, Dr.**
**Notburgaweg 6**
**D-4150 Krefeld-Traar(DE)**

(54) **Neue Aldehyde und deren Verwendung als Riechstoffe.**

(57) Gemische von Aldehyden der Formeln I und II

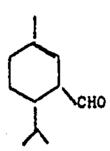 I) 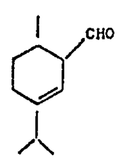 II)

wie sie bei der Hydroformulierung von α -Terpinen erhalten werden.

EP 0 011 273 A1

Düsseldorf, den 9. November 1979
Henkelstraße 67

HENKEL KGaA
ZR-FE/Patente
Dr.Gla/Se

P a t e n t a n m e l d u n g

D 5876

"Neue Aldehyde und deren Verwendung als Riechstoffe"

Es wurde gefunden, daß Gemische von Aldehyden der Formeln I und II,

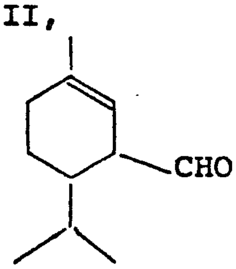

I

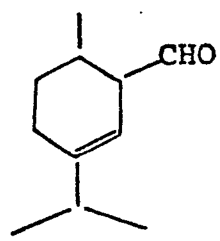

II

wie sie bei der Hydroformylierung von α-Terpinen erhalten werden, wertvolle neue Riechstoffe mit Salicylat- und Cumin-/Perilla-Note sowie außergewöhnlicher Haftfestigkeit darstellen.

Die Herstellung der neuen Verbindungsgemische erfolgt zweckmäßigerweise durch Umsetzung von α-Terpinen mit Kohlenmonoxid und Wasserstoff bei 70 - $160^{\circ}$ C und unter einem Druck von 100 - 400 bar. Als Katalysatoren werden dabei Gemische aus tertiären Phosphinen und diese tertiären Phosphine enthaltenden Rhodiumcarbonylkomplexen verwendet.

Als tertiäre Phosphine eignen sich Trialkylphosphine, deren Alkylreste 1 bis 20 Kohlenstoffatome aufweisen, sowie Triphenylphosphine, deren Phenylreste durch Alkyl- oder Alkoxygruppen mit 1 bis 4 Kohlenstoffatomen substituiert sein können, insbesondere jedoch Triphenylphosphin. In den Katalysatormischungen liegt die Molzahl von insgesamt vorhandenem Phosphin pro Grammatom Rhodium im Bereich von 20 bis 200.

Die genaue Zusammensetzung der katalytisch wirksamen Rhodiumcarbonylkomplexe ist nicht bekannt. Man kann jedoch davon ausgehen, daß es sich um Rhodiumkomplexe handelt, in denen ein oder mehrere Carbonylliganden durch Phosphinliganden ersetzt sind. Die tatsächlich wirksame Komplexverbindung wird in jedem Fall in situ unter den Bedingungen der Hydroformylierung gebildet. Die hierzu erforderliche Menge Rhodium kann deshalb dem Reaktionsgemisch in Form von Rhodiumchlorid, Rhodiumoxid, Rhodiumsalzen von Fettsäuren, Rhodiumchelaten, Rhodiumcarbonyl oder dimeren Rhodiumcarbonylchlorid zugeführt werden. Vorzugsweise werden Rhodiumkomplexe eingesetzt, die das im Katalysatorgemisch vorhandene Phosphin bereits als Ligand enthalten, beispielsweise die Verbindung $RhCl(CO)[P(C_6H_5)_3]_2$.

Die Rhodiumverbindungen setzt man vorteilhaft in solchen Mengen ein, daß, bezogen auf α-Terpinen, 5 bis 5 000 ppm, vorzugsweise 15 bis 400 ppm, berechnet als Metall, vorhanden sind.

Die Umsetzung kann in Abwesenheit von Lösungsmitteln durchgeführt werden. Es hat sich jedoch als zweckmäßig erwiesen, Lösungsmittel zu verwenden, wobei u.a. gesättigte Kohlenwasserstoffe wie Pentan, Hexan, Heptan, Cyclohexan, Benzol, Toluol oder Xylol, Ether wie Tetrahydrofuran und Dioxan, Alkohole wie Methanol, Ethanol und Isopropanol, oder Diole wie Ethylenglykol und Propylenglykol in Frage kommen. Bevorzugt wird die Hydroformylierung in gesättigten Kohlen-

/3

wasserstoffen oder Ethern durchgeführt.

Die Aufarbeitung des Reaktionsgemisches erfolgt auf dem Wege der Destillation.

Das bei der beschriebenen Hydroformylierung von α-Terpinen entstehende Reaktionsprodukt stellt ein Gemisch aus den Aldehyden der Formeln I und II dar. Das Gemisch hat Riechstoffeigenschaften und kann mit anderen Riechstoffen in verschiedenen Mengenverhältnissen zu neuen Riechstoffkompositionen gemischt werden. Im allgemeinen wird sich ihr Anteil in den Riechstoffkompositionen in den Mengen von 1 bis 50 Gewichtsprozent, bezogen auf die gesamte Komposition, bewegen. Derartige Kompositionen können direkt als Parfüm oder auch zur Parfümierung von Kosmetika, wie Cremes, Lotionen, Duftwässern, Aerosolen, Toiletteseifen usw. dienen. Sie können aber auch zur Geruchsverbesserung von technischen Produkten wie Wasch- und Reinigungsmitteln, Weichspülern, Textilbehandlungsmitteln eingesetzt werden. Zur Parfümierung der verschiedenen Produkte werden diesen die Kompositionen im allgemeinen in Konzentrationen von 0,05 bis 2 Gewichtsprozent zugesetzt.

/4

Beispiel:

In einem 5 l Hubrührautoklaven aus rostfreiem Stahl wurden 272 g (2 mol) α-Terpinen 3,8 g (14,5 mmol) $P(C_6H_5)_3$, 0,2 g (0,29 mmol) $RhCl(CO)[P(C_6H_5)_3]_2$ und 750 ml Tetrahydrofuran miteinander vermischt. Der Autoklav wurde mit Synthesegas gespült. Anschließend wurde ein aus gleichen Volumina Wasserstoff und Kohlenmonoxid bestehendes Gemisch bis zu einem Druck von 200 bar aufgepreßt. Der Autoklaveninhalt wurde unter Rühren auf 130° C aufgeheizt, anschließend 5 Stunden lang auf 130 - 140° C gehalten, dann auf Raumtemperatur abgekühlt. Aus dem Reaktionsgemisch wurde Tetrahydrofuran im Wasserstrahlvakuum abdestilliert. Bei der Destillation des Rückstandes im Ölpumpenvakuum gingen bei 98 - 100° C/20 mbar 225 g Produkt (68 % d. Th.) über.

Die gaschromatische Untersuchung zeigte, daß das Produkt ein Zweikomponentengemisch darstellt.

Carbonylzahl: 170 (theor. 168,7).

Das Produkt zeigte folgendes IR-Spektrum (Film):

3 005 $cm^{-1}$; 1 682 $cm^{-1}$ ($>C=C<_H$); 2 700 $cm^{-1}$; 1 725 $cm^{-1}$ (CHO); 1 380 $cm^{-1}$; 1 360 $cm^{-1}$; 845 $cm^{-1}$ (C=C tri-substituiert).

Geruch: Salicylat-Note, Cumin-Perilla-Note.

0011273

"Neue Aldehyde und deren Verwendung als Riechstoffe"

## Patentansprüche

1. Gemische von Aldehyden der Formeln I und II

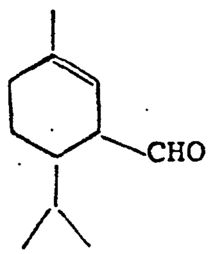 I)

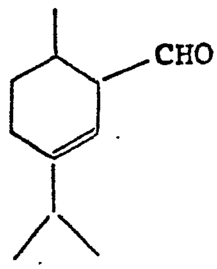 II)

wie sie bei der Hydroformylierung von α-Terpinen erhalten werden.

2. Verwendung der Aldehydgemische nach Anspruch 1 als Riechstoffe.

Europäisches Patentamt

# EUROPÄISCHER RECHERCHENBERICHT

0011273

Nummer der Anmeldung

EP 79 104 453.0

| EINSCHLÄGIGE DOKUMENTE | | | KLASSIFIKATION DER ANMELDUNG (Int.Cl.³) |
|---|---|---|---|
| Kategorie | Kennzeichnung des Dokuments mit Angabe, soweit erforderlich, der maßgeblichen Teile | betrifft Anspruch | |
| A | CH - A5 - 566 389 (N.V. CHEMISCHE FABRIEK "NAARDEN")<br>* Anspruch 1; Formel 10 *<br>---- | 1,2 | C 07 C 47/42<br>A 61 K 7/46<br>C 11 D 3/50<br>C 11 D 9/44<br>C 07 C 45/50 |
| | | | **RECHERCHIERTE SACHGEBIETE (Int. Cl.³)** |
| | | | A 61 K 7/46<br>A 61 L 9/00<br>C 07 C 45/50<br>C 07 C 47/42<br>C 11 D 3/50<br>C 11 D 9/44 |
| | | | **KATEGORIE DER GENANNTEN DOKUMENTE**<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| Berlin | 25-02-1980 | KNAACK |